# EUROPEAN PATENT SPECIFICATION

(11) **EP 2 654 789 B1**
(45) Date of publication and mention of the grant of the patent: **30.05.2018**
(21) Application number: 11801741.7
(22) Date of filing: 21.12.2011
(51) Int. Cl.: A61K 39/395, A61P 35/00, C07K 16/30, C07K 16/28

(54) **ANTIBODIES AGAINST HUMAN CD39 AND USE THEREOF**
ANTIKÖRPER GEGEN HUMANES CD39 UND IHRE VERWENDUNG
ANTICORPS CONTRE CD39 HUMAIN ET UTILISATION DE CEUX-CI

(30) Priority: 22.12.2010 US 201061426041 P
(43) Date of publication of application: 30.10.2013
(73) Proprietor: Orega Biotech, 69130 Ecully (FR); INSERM - Institut National de la Santé et de la Recherche Médicale, 75013 Paris (FR)
(72) Inventor: BENSUSSAN, Armand, F-75013 Paris (FR); BONNEFOY-BERARD, Nathalie, F-69003 Lyon (FR); ELIAOU, Jean-François, F-34980 Saint Clément de Rivière (FR); ALBERICI, Gilles, F-69290 Grezieu La Varenne (FR); BASTID, Jeremy, F-69290 Craponne (FR)
(74) Representative: Gallois, Valérie
(86) International application number: PCT/EP2011/073659
(87) International publication number: WO 2012/085132

(56) References cited:
- WO-A1-2009/095478
- DZHANDZHUGAZYAN KARINE N ET AL: "Ecto-ATP diphosphohydrolase/CD39 is overexpressed in differentiated human melanomas", FEBS LETTERS, vol. 430, no. 3, 3 July 1998 (1998-07-03), pages 227-230, XP002668791, ISSN: 0014-5793
- MEYER ET AL.: "Expression of cd39 and CD73 as means of evading anti-tumor immune responses in lung cancer", THE JOURNAL OF IMMUNOLOGY, vol. 184, 100.7, April 2010 (2010-04), XP002668792,
- JIN DACHUAN ET AL: "CD73 on Tumor Cells Impairs Antitumor T-Cell Responses: A Novel Mechanism of Tumor-Induced Immune Suppression", CANCER RESEARCH, vol. 70, no. 6, March 2010 (2010-03), XP002668793, ISSN: 0008-5472
- CLAYTON ALED ET AL: "Cancer Exosomes Express CD39 and CD73, Which Suppress T Cells through Adenosine Production", JOURNAL OF IMMUNOLOGY, vol. 187, no. 2, July 2011 (2011-07), pages 676-683, XP002668794, ISSN: 0022-1767
- J. BASTID ET AL: "Inhibition of CD39 Enzymatic Function at the Surface of Tumor Cells Alleviates Their Immunosuppressive Activity", CANCER IMMUNOLOGY RESEARCH, vol. 3, no. 3, 17 November 2014 (2014-11-17), pages 254-265, XP055340441, US ISSN: 2326-6066, DOI: 10.1158/2326-6066.CIR-14-0018

## Description

### FIELD OF THE INVENTION

The invention relates to antibodies against human CD39 for inhibiting the immunosuppressive effects of a CD39-expressing cancerous cell.

### BACKGROUND OF THE INVENTION

CD39 is an integral membrane protein with two transmembrane domains and a large extracellular region (Maliszewski et al, 1994) with nucleoside triphosphate diphosphohydrolase activity (Wang and Guidotti, 1996). In humans, CD39 is mainly expressed by regulatory T cells (Treg) but also other leukocytes. In cancers infiltrated with CD39 positive Tregs, CD39 plays a key role because it increases tumor angiogenesis and suppress the immune antitumor response by initiating the generation of adenosine (Stagg J et al, 2010).

To date, expression of CD39 by tumor cells has not been reported. Patent application WO2009/095478 describes the use of a CD39 antibody for the treatment or prevention of diseases, like cancers and infectious diseases, associated with an increased Treg activity.

However, certain cancerous pathologies are not associated with Treg activity. Thus, it would be very useful to elaborate methods and compositions for inhibiting the immunosuppressive effects of CD39-expressing cancerous cells.

WO2009/095478 relates to the use of anti-CD39 antibodies for treating diseases associated with an increased T regulatory activity.

The present disclosure aims to provide a method for inhibiting the immunosuppressive effects of CD39-expressing cancerous cells.

### SUMMARY OF THE INVENTION

The scope of the present invention is defined by the claims and any information that does not fall within the claims is provided for information only.

The present disclosure relates to a CD39 antibody for inhibiting the immunosuppressive effects of a CD39-expressing cancerous cell.

In particular, the disclosure relates to a CD39 antibody for the treatment or prevention of cancers. Preferably, said cancer is selected in the group consisting of melanoma, haematological cancer, ovarian cancer, thyroid cancer, lung cancer, kidney cancer.

The disclosure also relates to a method for treating cancers, said method comprising the step of administering to said subject an effective amount of a CD39 antagonist, preferably a CD39 monoclonal antibody inhibiting the immunosuppressive effect of CD39-expressing cancerous cells, said cancerous cells being preferably selected from the group consisting of haematological cancer cells, melanoma cells, ovarian cancer cells, thyroid cancer cells, lung cancer cells, and kidney cancer cells.

### DETAILED DESCRIPTION OF THE INVENTION

### Definitions

The term "CD39" denotes the CD39 protein also named as ectonucleoside triphosphate diphosphohydrolase-1 (ENTPD1). CD39 is an ectoenzyme that hydrolases ATP/UTP and ADP/UDP to the respective nucleosides such as AMP.

The term "CD39 antibody" refers to an antibody which binds to human CD39.

According to the present disclosure, "antibody" or "immunoglobulin" have the same meaning, and will be used equally in the present disclosure. The term "antibody" as used herein refers to immunoglobulin molecules and immunologically active portions of immunoglobulin molecules, i.e., molecules that contain an antigen binding site that specifically binds an antigen. As such, the term antibody encompasses not only whole antibody molecules, but also antibody fragments as well as variants (including derivatives) of antibodies and antibody fragments. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine the binding site specific to the antigenic epitope. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody binding site and the antigenic epitope. Antibody binding sites are made up of residues that are primarily from the hypervariable or complementarity determining regions (CDRs). Occasionally, residues from nonhypervariable or framework regions (FR) influence the overall domain structure and hence the binding site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs.

According to the disclosure, the term "chimeric antibody" refers to an antibody which comprises a VH domain and a VL domain of a CD39 antibody from any species, preferably mouse, and a CH domain and a CL domain of a human antibody.

According to the disclosure, the term "humanized antibody" refers to an antibody having variable region framework and constant regions from a human antibody but retains the CDRs of a CD39 antibody from any species, preferably mouse.

The term "Fab" denotes an antibody fragment having a molecular weight of about 50,000 and antigen binding activity, in which about a half of the N-terminal side of H chain and the entire L chain, among fragments obtained by treating IgG with a protease, papaine, are bound together through a disulfide bond.

The term "F(ab')2" refers to an antibody fragment having a molecular weight of about 100,000 and antigen binding activity, which is slightly larger than the Fab bound via a disulfide bond of the hinge region, among fragments obtained by treating IgG with a protease, pepsin.

The disclosure also encompasses molecules, preferably polypeptides, comprising the variable domain of the antibody of the disclosure, preferably the Fab of said antibody or the F(ab')2 of said antibody.

The term "Fab' " refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)2.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

By "purified" and "isolated" it is meant, when referring to a polypeptide (i.e. an antibody according to the disclosure) or to a nucleotide sequence, that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type are present. An "isolated" nucleic acid molecule which encodes a particular polypeptide refers to a nucleic acid molecule which is substantially free of other nucleic acid molecules that do not encode the polypeptide; however, the molecule may include some additional bases or moieties which do not deleteriously affect the basic characteristics of the composition.

In the context of the disclosure, the term "treating" or "treatment", as used herein, means reversing, alleviating, inhibiting the progress of, or preventing the disorder or condition to which such term applies, or one or more symptoms of such disorder or condition. A "therapeutically effective amount" is intended for a minimal amount of active agent (e.g., CD39 antibodies) which is necessary to impart therapeutic benefit to a subject. For example, a "therapeutically effective amount" to a mammal is such an amount which induces, ameliorates or otherwise causes an improvement in the pathological symptoms, disease progression or physiological conditions associated with or resistance to succumbing to a disorder.

As used herein, the term "prevention" refers to preventing the disease or condition from occurring in a subject which has not yet been diagnosed as having it.

As used herein, the term "subject" denotes a mammal, such as a rodent, a feline, a canine, and a primate. Preferably a subject according to the disclosure is a human.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. The terms "cancer" or "neoplasms" include malignancies of the various organ systems, such as affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. In the context of the present disclosure, the term "cancer" refers to melanoma, haematological cancer, ovarian cancer, thyroid cancer, lung cancer and kidney cancer.

As used herein, the term "haematological cancer" refers to cancers of blood and bone marrow, such as lymphoma, leukemia, and multiple myeloma. Preferably said haematological cancer is a lymphoma.

Leukemia refers to B-cell leukemia and T-cell leukemia and includes, but is not limited to, acute myeloid leukemia, chronic myeloid leukemia, acute lymphocytic leukemia (including, e.g., precursor B acute lymphoblastic leukemia, precursor T acute lymphoblastic leukemia, and acute biphenotypic leukemia), chronic lymphocytic leukemia (e.g., B-cell prolymphocytic leukemia), acute monocytic leukemia, acute myolegenous leukemia, chronic myelogenous leukemia, chronic granulocytic leukemia and T-cell prolymphocytic leukemia.

Lymphoma includes mantle cell lymphoma, Non-Hodgkin's lymphoma, and Hodgkin's lymphoma.

### Therapeutic uses of CD39 antibodies

Therefore, a first aspect of the disclosure provides methods and pharmaceutical compositions for inhibiting the immunosuppressive effects of a CD39-expressing cancerous cell.

The disclosure thus relates to a CD39 antagonist for use for inhibiting the immunosuppressive effects of a CD39-expressing cancerous cell.

The disclosure also relates to a method for inhibiting the immunosuppressive effects of a CD39-expressing cancerous cell which comprises the step of administering to a subject in need thereof a CD39 antagonist.

The disclosure also relates to a method for treating a subject suffering from a cancer, said method comprising the step of administering to said subject an effective amount of a CD39 antagonist, preferably a CD39 monoclonal antibody inhibiting the immunosuppressive effect of CD39-expressing cancerous cells, said cancerous cells being preferably selected from the group consisting of haematological cancer cells, melanoma cells, ovarian cancer cells, thyroid cancer cells, lung cancer cells, and kidney cancer cells.

The term "haematological cancer cells" refers to myeloid and/or lymphoid cells affected from cancer and includes lymphoma cells, leukemia cells and myeloma cells. Preferably, said haematological cancer cell is a lymphoma cell.

Examples of cancers to be treated include but are not limited to lung cancer, breast cancer, ovarian cancer, melanoma, skin carcinoma, liver cancer, gastric cancer, prostate cancer, pancreatic cancer, thyroid cancer, kidney cancer and lymphoma. Preferably, cancers to be treated are melanoma, haematological cancer, ovarian cancer, thyroid cancer, lung cancer and kidney cancer. Preferably, said haematological cancer is a lymphoma.

The CD39 antagonist is preferably selected in the group consisting of antibodies and chemical compounds, said antibodies and chemical compounds being able to down modulate the cell membrane expression of CD39 and/or to block or decrease CD39 ATPase/ADPase activity and/or to block or decrease cancer cells-mediated inhibition or suppression of the immune response. These CD39 antagonists significantly down modulate the cell membrane expression of CD39 and/or significantly block or decrease CD39 ATPase/ADPase activity and/or significantly block or decrease cancer cells-mediated inhibition or suppression of the immune response and/or to block or decrease cancer cells-mediated inhibition or suppression of the CD4 and/or CD8 T cell response.

The cell membrane expression of CD39 may be measured according to the protocol described in Examples 1 or 5 below.

The CD39 ATPase/ADPase activity may be measured according to the protocol described in Examples 3 or 7 below.

The cancer cells-mediated inhibition of the immune response may be measured according to the protocol described in Example 2 below.

The disclosure also relates to a method for identifying a subject suffering from a cancer comprising the step of determining the presence of CD39 expression on the cancerous cells of a sample from said subject, with the CD39 antibody according to the disclosure.

The present disclosure thus refers to the use of any CD39 antibody or fragment thereof, including CD39 chimeric antibodies (preferably chimeric mouse/human antibodies) or humanized CD39 antibodies, provided that said antibody inhibits the immunosuppressive effects of a CD39-expressing cancerous cell. Preferably, the CD39 antagonist is a CD39 monoclonal antibody.

Preferably, said CD39 monoclonal antibody is selected from the group consisting of BY12, BY40 and BA54g.

In a particular embodiment, said CD39 antibody is BY40. The inventors have indeed deposited a murine CD39 antibody (BY40) producing hybridoma at the Collection Nationale de Cultures de Microorganismes (CNCM, Institut Pasteur, 25 rue du Docteur Roux, 75724 Paris Cedex 15, France), in accordance with the terms of Budapest Treaty, on the 4th of January 2008. The deposited hybridoma has CNCM deposit number 1-3889. Said CD39 antibody may then be obtainable from the hybridoma deposited as CNCM-I-3889.

In another aspect, said CD39 antibody may comprise the VL chain of the antibody obtainable from hybridoma deposited as CNCM-I-3889 and the VH chain of the antibody obtainable from hybridoma deposited as CNCM-I-3889.

In another embodiment, said CD39 antibody may comprise a variable light chain (VL) comprising the CDRs of the VL chain of the antibody obtainable from hybridoma deposited as CNCM-I-3889 and a variable heavy chain (VH) comprising the CDRs of the VH chain of the antibody obtainable from hybridoma deposited as CNCM-I-3889.

In another aspect, the disclosure also encompasses molecules, preferably polypeptides, comprising the variable domain of the antibody obtainable from hybridoma deposited as CNCM-I-3889, preferably the Fab of said antibody or the F(ab')2 of said antibody.

In another embodiment of the invention, said CD39 antibody may comprise a heavy chain wherein the variable domain comprises at least one CDR having a sequence selected from the group consisting of SEQ ID NO:2 for CDR-H1, SEQ ID NO:3 for CDR-H2 and SEQ ID NO:4 for CDR-H3; and/or a light chain wherein the variable domain comprises at least one CDR having a sequence selected from the group consisting of SEQ ID NO:6 for CDR-L1, SEQ ID NO:7 for CDR-L2 and SEQ ID NO:8 for CDR-L3.

The inventors have cloned and characterized the variable domain of the light and heavy chains of said mAb BY40, and thus determined the complementarity determining regions (CDRs) domain of said antibody as described in Table 1:

**Table 1: VH, VL and CDR domains of mAb BY40:**

| **MAb BY40 Domains** | |
|---|---|
| VH | |
| VH CDR1 | GYTFT HYG (**SEQ ID NO:2**) |
| VH CDR2 | INTYT GEP (**SEQ ID NO:3**) |
| VH CDR3 | ARRRY EGNYV FYYFD YWGQG TTLTV SS **(SEQ ID NO:4)** |
| VL | |
| VL CDR1 | RASEN IYSYF S (**SEQ ID NO:6**) |
| VL CDR2 | TAKTL AE (**SEQ ID NO:7**) |
| VL CDR3 | QHHYV TPYTF GGGTK LEIKR (**SEQ ID NO:8**) |

An aspect of the disclosure relates to a CD39 antibody comprising a first heavy chain CDR sequence as set forth in SEQ ID NO:2, a second heavy chain CDR sequence as set forth in SEQ ID NO:3, and a third heavy chain CDR sequence as set forth in SEQ ID NO:4; and a first light chain CDR sequence as set forth in SEQ ID NO:6, a second light chain CDR sequence as set forth in SEQ ID NO:7, and a third light chain CDR sequence as set forth in SEQ ID NO:8. In a particular aspect, the heavy chain variable domain of said antibody has the amino acid sequence as set forth in SEQ ID NO: 1 and/or the light chain variable domain of said antibody has the amino acid sequence set forth in SEQ ID NO: 5.

Antibodies of the disclosure can be produced by any technique well known in the art. In particular said antibodies are produced by techniques as hereinafter described.

In another aspect, an antibody of the disclosure is a chimeric antibody, preferably a chimeric mouse/human antibody. In particular, said mouse/human chimeric antibody may comprise the variable domains of an antibody obtainable from hybridoma deposited as CNCM-I-3889.

An aspect of the disclosure relates to the hybridoma accessible under CNCM deposit number 1-3889.

In another aspect, an antibody of the disclosure is a humanized antibody. In particular, in said humanized antibody, the variable domain comprises human acceptor frameworks regions, and optionally human constant domain where present, and non-human donor CDRs, such as mouse CDRs as defined above.

The disclosure further provides fragments of said antibodies which include but are not limited to Fv, Fab, F(ab')2, Fab', dsFv, scFv, sc(Fv)2 and diabodies; and multispecific antibodies formed from antibody fragments.

In another aspect, the disclosure relates to a polypeptide which has a sequence selected from the group consisting of SEQ ID NO: 1, SEQ ID NO:2, SEQ ID NO:3, SEQ ID NO:4, SEQ ID NO:5; SEQ ID NO: 6; SEQ ID NO:7 and SEQ ID NO:8.

A further aspect of the disclosure relates to a nucleic acid sequence encoding an antibody of the invention or a fragment thereof.

In a particular aspect, the disclosure relates to a nucleic acid sequence encoding the VH domain of the antibody obtainable from hybridoma deposited as CNCM-I-3889 (BY40) or the VL domain of the antibody obtainable from hybridoma deposited as CNCM-I-3889 (BY40).

In a particular aspect, the disclosure relates to a nucleic acid sequence encoding the VH domain of mAb BY40 or the VL domain of mAb BY40.

**Table 2: Nucleic acids of VH and VL domains of mAb BY40:**

| | |
|---|---|
| VH domain: | |
| VL domain: | |

In a particular embodiment, said CD39 antibody is BA54g. The inventors have indeed deposited the BA54g antibody producing hybridoma at the Collection Nationale de Cultures de Microorganismes (CNCM, Institut Pasteur, 25 rue du Docteur Roux, 75724 Paris Cedex 15, France), in accordance with the terms of Budapest Treaty, on the 23^{rd} of June, 2009. The deposited hybridoma has CNCM deposit number CNCM 1-4171. Said CD39 antibody may then be obtainable from the hybridoma deposited under the accession number CNCM 1-4171.

In another aspect, said CD39 antibody may comprises the VL chain of the antibody obtainable from hybridoma deposited as CNCM-I-4171 and the VH chain of the antibody obtainable from hybridoma deposited as CNCM-I-4171.

In another embodiment, said CD39 antibody may comprise a variable light chain (VL) comprising the CDRs of the VL chain of the antibody obtainable from hybridoma deposited as CNCM-I-4171 and a variable heavy chain (VH) comprising the CDRs of the VH chain of the antibody obtainable from hybridoma deposited as CNCM-I-4171.

In another embodiment of the invention, said CD39 antibody may comprise a heavy chain wherein the variable domain comprises at least one CDR having a sequence selected from the group consisting of SEQ ID NO:12 for CDR-H1, SEQ ID NO:13 for CDR-H2 and SEQ ID NO:14 for CDR-H3; and/or a light chain wherein the variable domain comprises at least one CDR having a sequence selected from the group consisting of SEQ ID NO:16 for CDR-L1, SEQ ID NO:17 for CDR-L2 and SEQ ID NO: 18 for CDR-L3.

The inventors have cloned and characterized the variable domains of the light and heavy chains of said BA54g, and thus determined the complementarity determining regions (CDRs) of said antibody as described in Table 3.

**Table 3: VH, VL and CDR domains of mAb BA54g:**

| MAb BA54g Domains | |
|---|---|
| VH | |
| VH CDR1 | GGSRK LSCAA SGFTF SSFGM H (**SEQ ID NO:12**) |
| VH CDR2 | YISSG SSIIY YADTV KG (**SEQ ID NO: 13**) |
| VH CDR3 | WSTTV VATDY WGQGT TLTVS (**SEQ ID NO:14**) |
| VL | |
| VL CDR1 | KASEN VVTYV S (**SEQ ID NO:16**) |
| VL CDR2 | GASNR YT (**SEQ ID NO:17**) |
| VL CDR3 | CGQGY SYPYT FGGGT KLEIK R (**SEQ ID NO:18**) |

An aspect of the disclosure relates to a CD39 antibody comprising a first heavy chain CDR sequence as set forth in SEQ ID NO:12, a second heavy chain CDR sequence as set forth in SEQ ID NO:13, and a third heavy chain CDR sequence as set forth in SEQ ID NO:14; and a first light chain CDR sequence as set forth in SEQ ID NO:16, a second light chain CDR sequence as set forth in SEQ ID NO:17, and a third light chain CDR sequence as set forth in SEQ ID NO:18. In a particular embodiment, the heavy chain variable domain of said antibody has the amino acid sequence as set forth in SEQ ID NO: 11 and/or the light chain variable domain of said antibody has the amino acid sequence set forth in SEQ ID NO: 15.

Said antibodies can be produced by any technique well known in the art. In particular said antibodies are produced by techniques as hereinafter described.

According to an aspect, the antibody of the disclosure is a murine antibody.

In another aspect, the antibody of the disclosure is a chimeric antibody, preferably a chimeric mouse/human antibody. In particular, said mouse/human chimeric antibody may comprise the variable domains of an antibody obtainable from hybridoma deposited as CNCM 1-4171.

In another aspect, the antibody of the disclosure is a humanized antibody. In particular, in said humanized antibody, the variable domain comprises human acceptor frameworks regions, and optionally human constant domain where present, and non-human donor CDRs of an antibody obtainable from hybridoma deposited as CNCM I-4171.

The disclosure further provides for fragments of said antibodies which include but are not limited to Fv, Fab, F(ab')2, Fab', dsFv, scFv, sc(Fv)2 and diabodies; and multispecific antibodies formed from antibody fragments.

In another aspect, the disclosure relates to a polypeptide which has a sequence selected from the group consisting of SEQ ID NO: 11, SEQ ID NO:12, SEQ ID NO:13, SEQ ID NO:14, SEQ ID NO:15; SEQ ID NO: 16; SEQ ID NO:17 and SEQ ID NO:18.

In a particular aspect, the disclosure relates to a nucleic acid sequence encoding the VH domain of mAb BA54g or the VL domain of mAb BA54g, as detailed in Table 4.

In a particular aspect, the disclosure relates to a nucleic acid sequence encoding the VH domain of the antibody obtainable from hybridoma deposited as CNCM 1-4171 (SEQ ID NO:19) or the VL domain of the antibody obtainable from hybridoma deposited as CNCM 1-4171 (SEQ ID NO:20).

**Table 4: Nucleic acids of VH and VL domains of mAb BA54g:**

| | |
|---|---|
| VH domain: | |
| VL domain: | |

Typically, said nucleic acid is a DNA or RNA molecule, which may be included in any suitable vector, such as a plasmid, cosmid, episome, artificial chromosome, phage or a viral vector.

The terms "vector", "cloning vector" and "expression vector" mean the vehicle by which a DNA or RNA sequence (e.g. a foreign gene) can be introduced into a host cell, so as to transform the host and promote expression (e.g. transcription and translation) of the introduced sequence.

So, a further object of the disclosure relates to a vector comprising a nucleic acid of the invention.

Such vectors may comprise regulatory elements, such as a promoter, enhancer, terminator and the like, to cause or direct expression of said antibody upon administration to a subject. Examples of promoters and enhancers used in the expression vector for animal cell include early promoter and enhancer of SV40 (Mizukami T. et al. 1987), LTR promoter and enhancer of Moloney mouse leukemia virus (Kuwana Y et al. 1987), promoter (Mason JO et al. 1985) and enhancer (Gillies SD et al. 1983) of immunoglobulin H chain and the like.

Any expression vector for animal cell can be used, so long as a gene encoding the human antibody C region can be inserted and expressed. Examples of suitable vectors include pAGE107 (Miyaji H et al. 1990), pAGE103 (Mizukami T et al. 1987), pHSG274 (Brady G et al. 1984), pKCR (O'Hare K et al. 1981), pSG1 beta d2-4-(Miyaji H et al. 1990) and the like.

Other examples of plasmids include replicating plasmids comprising an origin of replication, or integrative plasmids, such as for instance pUC, pcDNA, pBR, and the like.

Other examples of viral vector include adenoviral, retroviral, herpes virus and AAV vectors. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO 95/14785, WO 96/22378, US 5,882,877, US 6,013,516, US 4,861,719, US 5,278,056 and WO 94/19478.

A further object of the present disclosure relates to a cell which has been transfected, infected or transformed by a nucleic acid and/or a vector according to the disclosure.

The term "transformation" means the introduction of a "foreign" (i.e. extrinsic or extracellular) gene, DNA or RNA sequence to a host cell, so that the host cell will express the introduced gene or sequence to produce a desired substance, typically a protein or enzyme coded by the introduced gene or sequence. A host cell that receives and expresses introduced DNA or RNA bas been "transformed".

The nucleic acids of the disclosure may be used to produce an antibody of the disclosure in a suitable expression system. The term "expression system" means a host cell and compatible vector under suitable conditions, e.g. for the expression of a protein coded for by foreign DNA carried by the vector and introduced to the host cell.

Common expression systems include E. coli host cells and plasmid vectors, insect host cells and Baculovirus vectors, and mammalian host cells and vectors. Other examples of host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from lymphoblasts, fibroblasts, embryonic cells, epithelial cells, nervous cells, adipocytes, etc.). Examples also include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is defective (Urlaub G et al; 1980), rat YB2/3HL.P2.G11.16Ag.20 cell (ATCC CRL1662, hereinafter referred to as "YB2/0 cell"), and the like.

The present disclosure also relates to a method of producing a recombinant host cell expressing an antibody according to the disclosure, said method comprising the steps of: (i) introducing in vitro or ex vivo a recombinant nucleic acid or a vector as described above into a competent host cell, (ii) culturing in vitro or ex vivo the recombinant host cell obtained and (iii), optionally, selecting the cells which express and/or secrete said antibody. Such recombinant host cells can be used for the production of antibodies of the disclosure.

CD39 antibodies may be produced by any technique known in the art, such as, without limitation, any chemical, biological, genetic or enzymatic technique, either alone or in combination.

For example, CD39 antibodies can be raised according to known methods by administering the appropriate antigen or epitope to a host animal selected, e.g., from pigs, cows, horses, rabbits, goats, sheep, and mice, among others.

Various adjuvants known in the art can be used to enhance antibody production. Although antibodies useful in practicing the disclosure can be polyclonal, monoclonal antibodies are preferred. Monoclonal antibodies against CD39 can be prepared and isolated using any technique that provides for the production of antibody molecules by continuous cell lines in culture.

Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).

Knowing the amino acid sequence of the desired antibody, one skilled in the art can readily produce said antibodies, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions. Alternatively, CD39 antibodies can be synthesized by recombinant DNA techniques well-known in the art. For example, antibodies can be obtained as DNA expression products after incorporation of DNA sequences encoding the antibodies into expression vectors and introduction of such vectors into suitable eukaryotic or prokaryotic hosts that will express the desired antibodies, from which they can be later isolated using well-known techniques.

In particular, the disclosure further relates to a method of producing an antibody of the disclosure, which method comprises the steps consisting of: (i) culturing a cell expressing a CD39 antibody under conditions suitable to allow expression of said antibody; and (ii) recovering the expressed antibody.

In another particular aspect, the method comprises the steps of:
(i) culturing an hybridoma expressing a CD39 antibody, (e.g. the hybridoma deposited as CNCM-I-3889 or CNCM-I-4171), under conditions suitable to allow expression of antibody; and
(ii) recovering the expressed antibody.

CD39 antibodies are suitably separated from the culture medium by conventional immunoglobulin purification procedures such as, for example, protein A-Sepharose, hydroxylapatite chromatography, gel electrophoresis, dialysis, or affinity chromatography.

In a particular aspect, a human chimeric CD39 antibody can be produced by obtaining nucleic sequences encoding VL and VH domains as previously described, constructing a human chimeric antibody expression vector by inserting them into an expression vector for animal cell having genes encoding human antibody CH and human antibody CL, and expressing the coding sequence by introducing the expression vector into an animal cell.

As the CH domain of a human chimeric antibody, it may be any region which belongs to human immunoglobulin, but those of IgG class are suitable and any one of subclasses belonging to IgG class, such as IgG1, IgG2, IgG3 and IgG4, can also be used. Also, as the CL of a human chimeric antibody, it may be any region which belongs to Ig, and those of kappa class or lambda class can be used.

Methods for producing chimeric antibodies involve conventional recombinant DNA and gene transfection techniques are well known in the art (See Morrison SL. et al. (1984) and patent documents US5,202,238; and US5,204, 244).

A CD39 humanized antibody may be produced by obtaining nucleic acid sequences encoding CDR domains, as previously described, constructing a humanized antibody expression vector by inserting them into an expression vector for animal cell having genes encoding (i) a heavy chain constant region identical to that of a human antibody and (ii) a light chain constant region identical to that of a human antibody, and expressing the genes by introducing the expression vector into an animal cell.

The humanized antibody expression vector may be either of a type in which a gene encoding an antibody heavy chain and a gene encoding an antibody light chain exists on separate vectors or of a type in which both genes exist on the same vector (tandem type). In respect of easiness of construction of a humanized antibody expression vector, easiness of introduction into animal cells, and balance between the expression levels of antibody H and L chains in animal cells, humanized antibody expression vector of the tandem type is preferred (Shitara K et al. 1994). Examples of tandem type humanized antibody expression vector include pKANTEX93 (WO 97/10354), pEE18 and the like.

Methods for producing humanized antibodies based on conventional recombinant DNA and gene transfection techniques are well known in the art (See, e. g., Riechmann L. et al. 1988; Neuberger MS. et al. 1985). Antibodies can be humanized using a variety of techniques known in the art including, for example, CDR-grafting (EP 239,400; PCT publication WO91/09967; U.S. Pat. Nos. 5,225,539; 5,530,101; and 5,585,089), veneering or resurfacing (EP 592,106; EP 519,596; Padlan EA (1991); Studnicka GM et al. (1994); Roguska MA. et al. (1994)), and chain shuffling (U.S. Pat. No.5,565,332). The general recombinant DNA technology for preparation of such antibodies is also known (see European Patent Application EP 125023 and International Patent Application WO 96/02576).

Fab can be obtained by treating an antibody which specifically reacts with human CD39 with a protease, papaine. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

F(ab')2 can be obtained treating an antibody which specifically reacts with human CD39 with a protease, pepsin. Also, the F(ab')2 can be produced by binding Fab' described below via a thioether bond or a disulfide bond.

Fab' can be obtained treating F(ab')2 which specifically reacts with human CD39 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

scFv can be produced by obtaining cDNA encoding the VH and VL domains as previously described, constructing DNA encoding scFv, inserting the DNA into an expression vector for prokaryote, or an expression vector for eukaryote, and then introducing the expression vector into a prokaryote or eukaryote (as appropriate) to express the scFv. To generate a humanized scFv fragment, a well known technology called CDR grafting may be used, which involves selecting the complementary determining regions (CDRs) from a donor scFv fragment, and grafting them onto a human scFv fragment framework of known three dimensional structure (see, e. g., WO98/45322; WO 87/02671; US5,859,205; US5,585,089; US4,816,567; EP0173494).

Amino acid sequence modification(s) of the antibodies described herein are contemplated. For example, it may be desirable to improve the binding affinity and/or other biological properties of the antibody. It is known that when a humanized antibody is produced by simply grafting only CDRs in VH and VL of an antibody derived from a non-human animal in FRs of the VH and VL of a human antibody, the antigen binding activity is reduced in comparison with that of the original antibody derived from a non-human animal. It is considered that several amino acid residues of the VH and VL of the non-human antibody, not only in CDRs but also in FRs, are directly or indirectly associated with the antigen binding activity. Hence, substitution of these amino acid residues with different amino acid residues derived from FRs of the VH and VL of the human antibody would reduce of the binding activity. In order to resolve the problem, in antibodies grafted with human CDR, attempts have to be made to identify, among amino acid sequences of the FR of the VH and VL of human antibodies, an amino acid residue which is directly associated with binding to the antibody, or which interacts with an amino acid residue of CDR, or which maintains the three-dimensional structure of the antibody and which is directly associated with binding to the antigen. The reduced antigen binding activity could be increased by replacing the identified amino acids with amino acid residues of the original antibody derived from a non-human animal.

Modifications and changes may be made in the structure of the antibodies of the present disclosure, and in the DNA sequences encoding them, and still obtain a functional molecule that encodes an antibody with desirable characteristics.

In making the changes in the amino sequences, the hydropathic index of amino acids may be considered. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

A further object of the present disclosure also encompasses function-conservative variants of the antibodies of the present disclosure.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, more preferably at least 85%, still preferably at least 90 %, and even more preferably at least 95%, and which has the same or substantially similar properties or functions as the native or parent protein to which it is compared.

Two amino acid sequences are "substantially homologous" or "substantially similar" when greater than 80 %, preferably greater than 85 %, preferably greater than 90 % of the amino acids are identical, or greater than about 90 %, preferably grater than 95 %, are similar (functionally identical) over the whole length of the shorter sequence. Preferably, the similar or homologous sequences are identified by alignment using, for example, the GCG (Genetics Computer Group, Program Manual for the GCG Package, Version 7, Madison, Wisconsin) pileup program, or any of sequence comparison algorithms such as BLAST, FASTA, etc.

For example, certain amino acids may be substituted by other amino acids in a protein structure without appreciable loss of activity. Since the interactive capacity and nature of a protein define the protein's biological functional activity, certain amino acid substitutions can be made in a protein sequence, and, of course, in its DNA encoding sequence, while nevertheless obtaining a protein with like properties. It is thus contemplated that various changes may be made in the antibodies sequences of the disclosure, or corresponding DNA sequences which encode said antibodies, without appreciable loss of their biological activity.

It is known in the art that certain amino acids may be substituted by other amino acids having a similar hydropathic index or score and still result in a protein with similar biological activity, i.e. still obtain a biological functionally equivalent protein.

As outlined above, amino acid substitutions are generally therefore based on the relative similarity of the amino acid side-chain substituents, for example, their hydrophobicity, hydrophilicity, charge, size, and the like. Exemplary substitutions which take various of the foregoing characteristics into consideration are well known to those of skill in the art and include: arginine and lysine; glutamate and aspartate; serine and threonine; glutamine and asparagine; and valine, leucine and isoleucine.

Another type of amino acid modification of the antibody of the disclosure may be useful for altering the original glycosylation pattern of the antibody.

By "altering" is meant deleting one or more carbohydrate moieties found in the antibody, and/or adding one or more glycosylation sites that are not present in the antibody.

Glycosylation of antibodies is typically N-linked. "N-linked" refers to the attachment of the carbohydrate moiety to the side chain of an asparagine residue. The tripeptide sequences asparagine-X-serine and asparagines-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites).

Another type of covalent modification involves chemically or enzymatically coupling glycosides to the antibody. These procedures are advantageous in that they do not require production of the antibody in a host cell that has glycosylation capabilities for N-or O-linked glycosylation. Depending on the coupling mode used, the sugar(s) may be attached to (a) arginine and histidine, (b) free carboxyl groups, (c) free sulfhydryl groups such as thoseof cysteine, (d) free hydroxyl groups such as those of serine, threonine, orhydroxyproline, (e) aromatic residues such as those of phenylalanine, tyrosine, or tryptophan, or (f) the amide group of glutamine. For example, such methods are described in WO87/05330.

Removal of any carbohydrate moieties present on the antibody may be accomplished chemically or enzymatically. Chemical deglycosylation requires exposure of the antibody to the compound trifluoromethanesulfonic acid, or an equivalent compound. This treatment results in the cleavage of most or all sugars except the linking sugar (N-acetylglucosamine or N-acetylgalactosamine), while leaving the antibody intact. Chemical deglycosylation is described by Sojahr H. et al. (1987) and by Edge, AS. et al. (1981). Enzymatic cleavage of carbohydrate moieties on antibodies can be achieved by the use of a variety of endo-and exo-glycosidases as described by Thotakura, NR. et al. (1987).

Another type of covalent modification of the antibody comprises linking the antibody to one of a variety of non proteinaceous polymers, eg. , polyethylene glycol, polypropylene glycol, or polyoxyalkylenes, in the manner set forth in US Patent Nos. 4,640, 835; 4,496, 689; 4,301, 144; 4,670, 417; 4,791, 192 or 4,179,337.

It may be also desirable to modify the antibody of the disclosure with respect to effector function, e.g. so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing inter-chain disulfide bond formation in this region. The homodimeric antibody thus generated may have improved internalization capability and/or increased complement-mediated cell killing and/or antibody-dependent cellular cytotoxicity (ADCC) (Caron PC. et al. 1992; and Shopes B. 1992)

### Pharmaceutical compositions

The disclosure also relates to pharmaceutical composition comprising CD39 antagonists for inhibiting the immunosuppressive effects of a CD39-expressing cancerous cell.

Therefore, CD39 antagonists, particularly CD39 antibodies, may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semI-3889solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the antibody may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

A CD39 antagonist can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

CD39 antibodies may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, e.g. tablets or other solids for oral administration; time release capsules; and any other form currently used.

In certain aspects, the use of liposomes and/or nanoparticles is contemplated for the introduction of antibodies into host cells. The formation and use of liposomes and/or nanoparticles are known to those of skill in the art.

Nanocapsules can generally entrap compounds in a stable and reproducible way. To avoid side effects due to intracellular polymeric overloading, such ultrafine particles (sized around 0.1 µm) are generally designed using polymers able to be degraded in vivo. Biodegradable polyalkyl-cyanoacrylate nanoparticles that meet these requirements are contemplated for use in the present disclosure, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The physical characteristics of liposomes depend on pH, ionic strength and the presence of divalent cations.

Antibodies of the disclosure can be produced and/or modified by any technique known in the art, as above described.

### FIGURES:

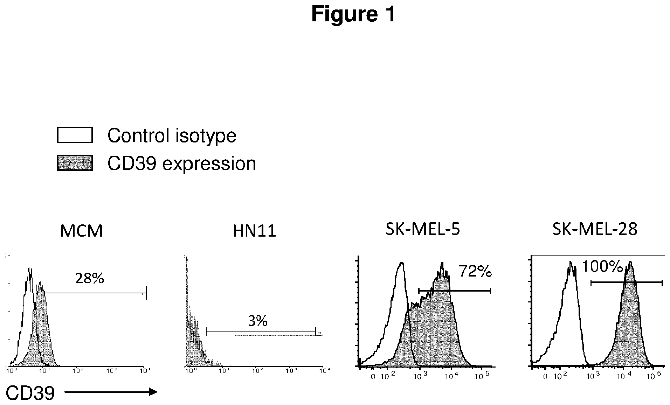

**Figure 1****: Expression of CD39 by melanoma cells.** Melanoma cell lines (HN11, SK-MEL-5 and SK-MEL-28) and melanoma cells from a primary tumor (MCM) were stained with CD39 antibody (BA54g or clone A1, Ebioscience) and CD39 expression was analyzed by flow cytometry. The percentage of CD39 positive tumor cells within the population is indicated. MCM, SK-MEL-5 and SK-MEL-28 express CD39 at their cell surface.

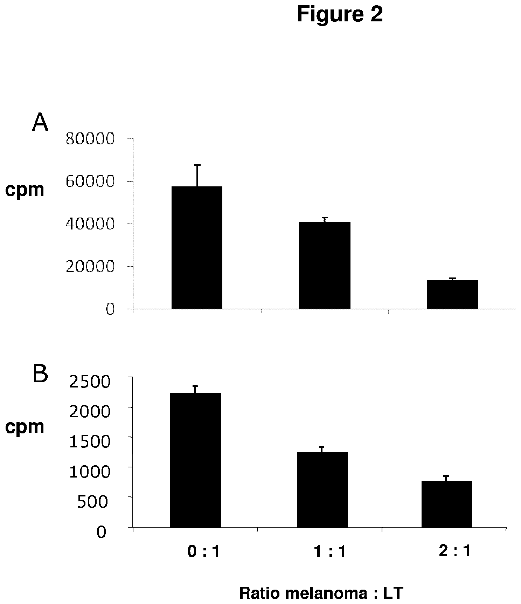

**Figure 2****: Immunosuppressive activity of melanoma cells.** 1x10⁵ activated CD8 (**A**) or activated CD4 (**B**) T cells from healthy donors' blood were incubated alone or in the presence of melanoma cell line (SK-MEL-5) at 1:1 or 2:1 ratios (SK-MEL-5:T cells). Anti-CD3 mAb (1 µg/mL, clone UCHT1) was previously immobilized on cell culture plates (T cell activation). Anti-CD28 mAb (1 µg/mL, clone CD28.2) was added at the beginning of the culture. After 4 days of culture, incorporation of thymidine (18h) was performed to measure CD8 and CD4 T cells' proliferation. Each condition was made in triplicate.

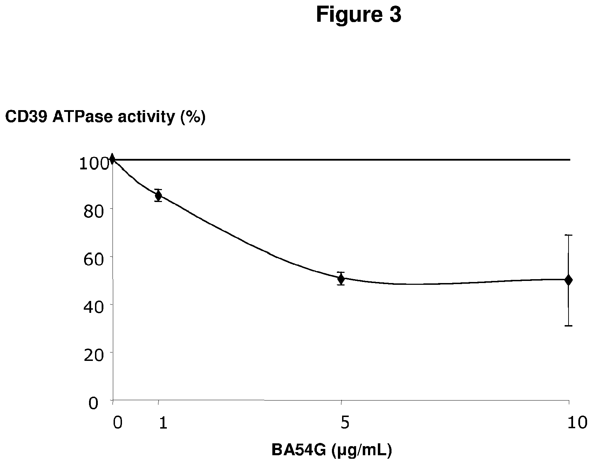

**Figure 3****: CD39 (BA54g) antibody inhibits CD39 ATPase activity of melanoma cells.** 1x10⁵ melanoma cells (SK-MEL-5) were cultured in complete RPMI medium alone or in presence of BA54g mAb for 16h. Cells were then washed three times in phosphate free buffer (10 mM glucose, 20 mM Hepes, 5 mM KCl, 120 mM NaCl, 2 mM CaCl2) and resuspended in the same buffer supplemented with 100 µM ATP for 15 min. Phosphate concentration in supernatants was measured after addition of Malachite green/polyvinylalcohol /ammonium mobylate solution for 20 min by a spectrophotometer at 620 nm and compared against a standard curve. Experiment was performed in triplicate.

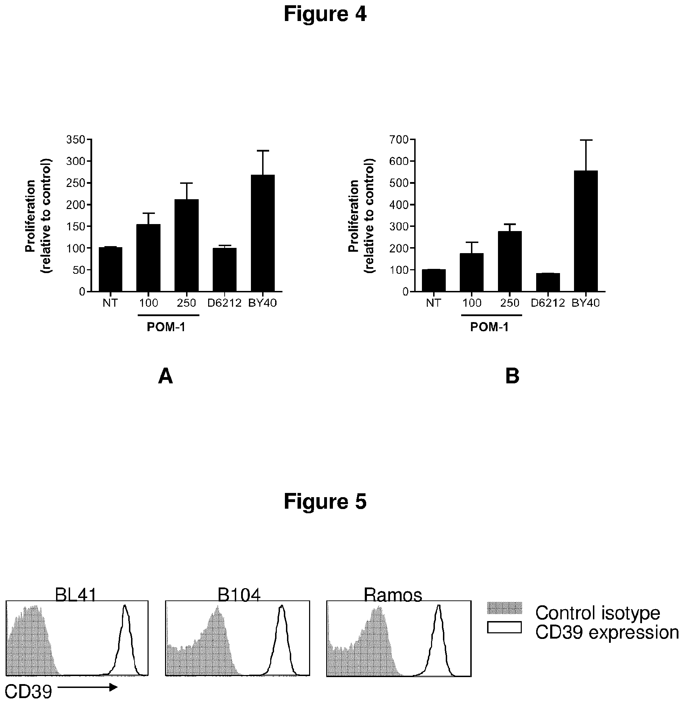

**Figure 4** **: CD39 antibody (BY40) restores T cell responses against melanoma cells.** 5x10⁴ CFSE-labelled PBMC were cultured in the presence of 5x10³ irradiated (100 Gy) SK-MEL-5 (NT). POM-1 (100 or 250 µM), BY40 or control isotype antibody D6212 (5 µg/mL) were added at the beginning of the culture and after 2 days of culture. After 5 days of culture, proliferation of CFSE-labelled CD4 (**A**) and CD8 (**B**) T cells was analysed by flow cytometry. Experiment was performed in duplicate and results are representative of 2 independent experiments.
**Figure 5****: Expression of CD39 by lymphoma cells.** Lymphoma cell lines (BL41, burkitt lymphoma; B104, B cell lymphoma; Ramos, Burkitt lymphoma) were stained with CD39 antibody (clone A1; Ebioscience) and CD39 expression was analyzed by flow cytometry. Results are representative of four experiments. BL41, B104 and Ramos express CD39 at their cell surface.

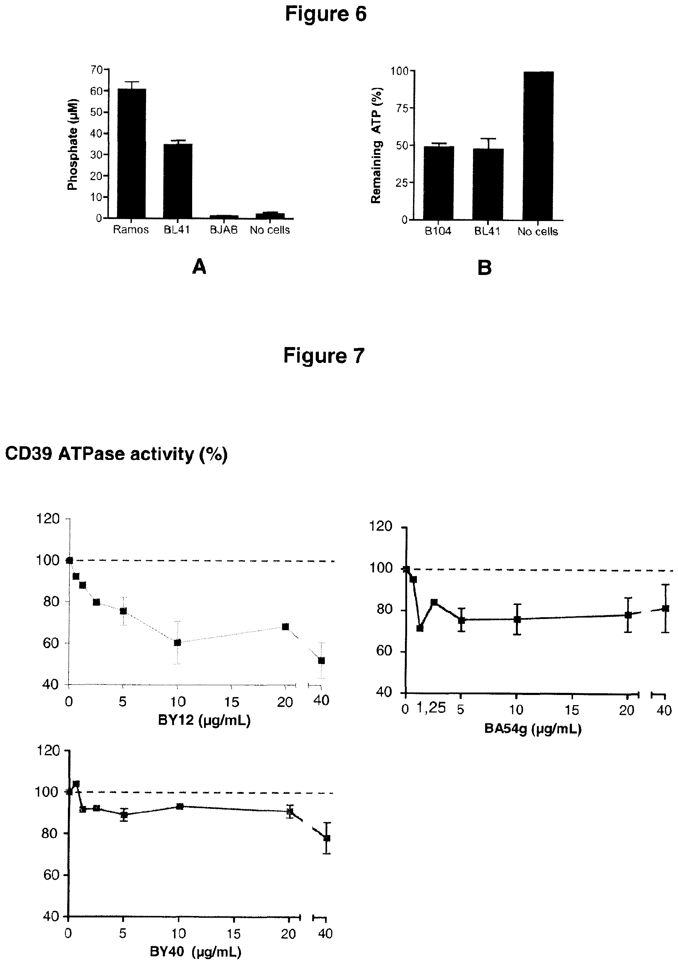

**Figure 6** **: ATPAse activity of lymphoma cells. (A)** 5x10⁴ lymphoma cells expressing CD39 (Ramos, BL41) or not (BJAB) were cultured in phosphate free buffer supplemented with 100 µM ATP for 15 min. Phosphate concentration in supernatants was measured after addition of Malachite green/polyvinylalcohol/ammonium mobylate solution for 10 min using a spectrophotometer at 620 nm. Experiment was performed in triplicate. (**B**) 5x10⁴ lymphoma cells expressing CD39 (BL41 and B104) were cultured in complete RPMI medium for 30 min with 10 µM ATP. The concentration of "unhydrolysed" extracellular ATP was determined using the ATPlite luminescence ATP Detection Assay System (Perkin Elmer). Results are the mean of 2 independent experiments.
**Figure 7****: CD39 antibodies inhibit CD39 ATPase activity of lymphoma cells.** 1x10⁵ lymphoma cells (Ramos) were cultured in complete RPMI medium alone or in presence of BA54g, BY12 or BY40 mAbs, at the indicated concentrations, for 16h. Cells were then washed three times in phosphate free buffer (10 mM glucose, 20 mM Hepes, 5 mM KCl, 120 mM NaCl, 2 mM CaCl2) and resuspended in the same buffer supplemented with 100 µM ATP for 15 min. Phosphate concentration in supernatants was measured after addition of Malachite green/polyvinylalcohol /ammonium mobylate solution for 20 min by a spectrophotometer at 620 nm and compared against a standard curve. Results are the mean of two independent experiments.

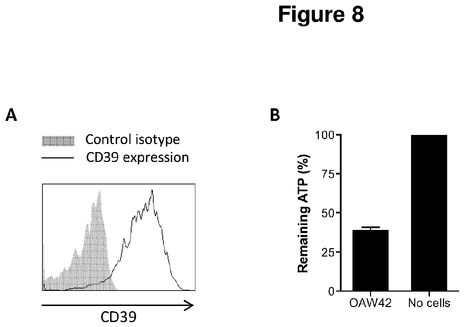

**Figure 8** **: Expression and activity of CD39 on ovarian cancer cells. (A)** Ovarian cancer cells (OAW42) were stained with CD39 antibody (clone A1, Ebiosciences) and CD39 expression was analysed by flow cytometry. Results are representative of two independent experiments. (**B**) 5x10⁴ OAW42 cells were cultured in complete RPMI medium for 30 min with 10 µM ATP. The concentration of "unhydrolysed" extracellular ATP was determined using the ATPlite luminescence ATP Detection Assay System.(Perkin Elmer) Experiment was performed in triplicate.

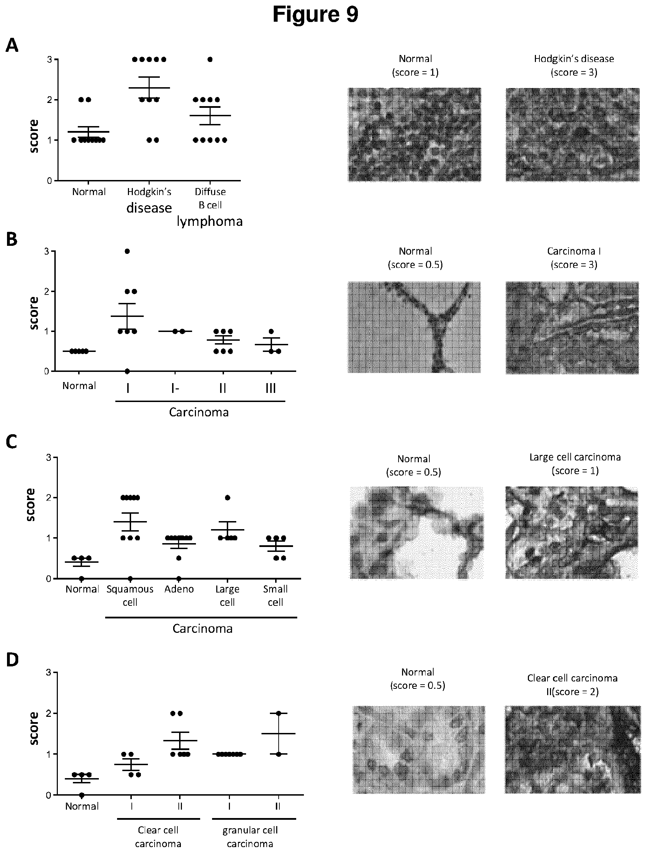

**Figure 9** **: CD39 Expression on a panel of normal and cancer tissues.** Multiple organ cancer and normal tissue Microarray (MC5002) from US Biomax, Inc. were used to analyze expressions of CD39. MC5002 contained 500 cores with 18 of most common types of cancer (20 -25 cases/type) and normal controls (5 cases/type). The tissues were formalin fixed, paraffin embedded. Array sections were mounted on the positive charged Fisher SuperFrost glass slide which is suitable for antigen detection. Two CD39 antibodies were evaluated for IHC suitability and the CD39 antibody from Abcam (clone 22A9) were selected. IHC staining was then performed using this CD39 antibody at 1.52 µg/mL (clone 22A9, Abcam). Manual scoring of intensity of staining was performed. The intensity (Strength, 0-3) of CD39 staining was scored as negative (0), possible positive (0.5), weak (1), moderate (2), or strong (3). Results for normal or cancer tissues from lymph node (**A**), thyroid (**B**), lung (**C**) or kidney (**D**) are shown. Each dot represents one sample.

### EXAMPLES

### Example 1: Expression of CD39 by melanoma cells

Protocol: Melanoma cell lines (HN11, SK-MEL-5 and SK-MEL-28) and melanoma cells from a primary tumor (MCM) were stained with CD39 antibody (BA54g or clone A1 from Ebioscience) and CD39 expression was analyzed by flow cytometry. The percentage of CD39 positive tumor cells within the population was then evaluated.

Results: **CD39 is expressed by melanoma tumor cells.** CD39 is an ectonucleotidase that converts extra-cellular ATP generated in sites of immune activation, leading to adenosine generation, an inhibitor of cell proliferation. It has been reported that CD39 is expressed at the cell surface of human Tregs as well as other leukocytes (Stagg J, Oncogene 2010) but not at the surface of tumor cells. Here we used the CD39 antibody BA54g as well as a commercially available one (clone A1 from Ebioscience) to examine human CD39 expression at the cell surface of human melanoma cells. As shown in figure 1, melanoma cells from a human primary tumor (MCM) and from SK-MEL-5 and SK-MEL-28 cell lines display membranous expression of CD39, while another cell line (HN11) is negative for CD39. Thus, melanoma tumor cells can express CD39 at their cell surface.

### Example 2: Activity of CD39 positive melanoma cells towards effector T cells

Protocol: 1x10⁵ activated CD8 (**A**) or activated CD4 (**B**) T cells from healthy donors' blood were incubated alone or in the presence of melanoma cell line (SK-MEL-5) at 1:1 or 2:1 ratios (SK-MEL-5:T cells). Anti-CD3 mAb (1 µg/mL, clone UCHT1) was previously immobilized on cell culture plates (T cell activation). Anti-CD28 mAb (1 µg/mL, clone CD28.2) was added at the beginning of the culture. After 4 days of culture, incorporation of thymidine (18h) was performed to measure CD8 and CD4 T cells' proliferation. Each condition was made in triplicate.

Results: **CD39 positive melanoma cells are suppressive towards CD4 and CD8 effector T cells.** As CD39 participates to the generation of adenosine that is a potent immunosuppressor, we speculated that CD39 positive melanoma cells would inhibit CD4 and CD8 activated T cell proliferation. As shown in figure 2, SK-MEL-5 cells indeed inhibit CD4 and CD8 effector T cells proliferation in a dose dependant manner. Thus, CD39 positive melanoma cells exert a potent immunosuppressive activity which might affect the antitumor response *in vivo.*

### Example 3: Inhibition of melanoma-CD39 enzymatic activity by BA54g

Protocol: 1x10⁵ melanoma cells (SK-MEL-5) were cultured in complete RPMI medium alone or in presence of BA54g mAb for 16h. Cells were then washed three times in phosphate free buffer (10 mM glucose, 20 mM Hepes, 5 mM KCl, 120 mM NaCl, 2 mM CaCl2) and resuspended in the same buffer supplemented with 100 µM ATP for 15 min. Phosphate concentration in supernatants was measured after addition of Malachite green/polyvinylalcohol /ammonium mobylate solution for 20 min by a spectrophotometer at 620 nm and compared against a standard curve. Experiment was performed in triplicate.

Results: **Inhibition of melanoma-CD39 enzymatic activity by CD39 monoclonal antibody (mAb) BA54g.** In order to alleviate the adenosine-mediated immunosuppression by melanoma cells, we cultured CD39 positive SK-MEL-5 cells in the presence of CD39 mAb BA54g and measured CD39 enzymatic activity. As shown in figure 3, BA54g inhibits CD39 enzymatic activity in a dose dependant manner, suggesting that CD39 mAbs might be useful tools to abrogate the generation of adenosine by melanoma cells and to restore an efficient antitumor response.

### Example 4: Inhibition of melanoma cell -mediated suppression by BY40

Protocol: 5x10⁴ CFSE-labelled PBMC were cultured in the presence of 5x10³ irradiated (100 Gy) SK-MEL-5 (NT). POM-1 (100 or 250 µM), BY40 or control isotype antibody D6212 (5 µg/mL) were added at the beginning of the culture and after 2 days of culture. After 5 days of culture, proliferation of CFSE-labelled CD4 (**A**) and CD8 (**B**) T cells was analysed by flow cytometry. Experiment was performed in duplicate and results are representative of 2 independent experiments.

Results: **CD39 antibody (BY40) restores T cell responses against melanoma cells.** CD39 positive melanoma cells are suppressive towards CD4 and CD8 T cells. As CD39 blocking antibodies inhibit CD39 enzymatic activity (figure 3), we analysed whether inhibition of CD39 at the cell surface of melanoma cells restores T cell proliferation. For that purpose, we co-cultured T lymphocytes and irradiated melanoma cells in the presence of CD39 mAb BY40 or a synthetic inhibitor of CD39, POM-1 (sodium metatungstate). As shown in figure 4, BY40 and POM-1 increased CD4 and CD8 T cell proliferation. Thus, inhibition of effector T cells by CD39-expressing melanoma cells can be abrogated by CD39 blocking mAbs.

### Example 5: Expression of CD39 by lymphoma tumor cells

Protocol: Lymphoma cell lines (BL41, burkitt lymphoma; B104, B cell lymphoma; Ramos, Burkitt lymphoma) were stained with CD39 antibody (clone A1; Ebioscience) and CD39 expression was analyzed by flow cytometry. Results are representative of four experiments.

Results: **CD39 is expressed by lymphoma tumor cells.** In order to assess whether lymphoma cells might also express CD39, we measured CD39 expression at the cell surface of various lymphoma tumor cell lines by flow cytometry using the CD39 antibody A1 (Ebioscience). As shown in figure 5, the Burkitt lymphoma cell lines BL41 and Ramos as well as the B cell lymphoma cell line B104 all express CD39 at their cell surface. Thus, lymphoma cells can express CD39 which might participate to suppress the immune system response.

### Example 6: ATPase activity of lymphoma cells

Protocol: (**A**) 5x10⁴ lymphoma cells expressing CD39 (Ramos, BL41) or not (BJAB) were cultured in phosphate free buffer supplemented with 100 µM ATP for 15 min. Phosphate concentration in supernatants was measured after addition of Malachite green/polyvinylalcohol/ammonium mobylate solution for 10 min using a spectrophotometer at 620 nm. Experiment was performed in triplicate. (B) 5x10⁴ lymphoma cells expressing CD39 (BL41 and B104) were cultured in complete RPMI medium for 30 min with 10 µM ATP. The concentration of "unhydrolysed" extracellular ATP was determined using the ATPlite luminescence ATP Detection Assay System (Perkin Elmer).

Results: **CD39 positive lymphoma cells, but not CD39 negative lymphoma cells, exert an ATPase activity.** In order to assess the enzymatic activity of CD39 at the surface of Ramos, BL41 and B104 cell lines, we measured their capacity to metabolize ATP, either by measuring phosphate concentration in cell supernatant or by measuring the remaining ATP. As shown in figure 6, the CD39 positive cell lines (Ramos, BL41 and B104), but not the negative one (BJAB), had the ability to hydrolyse ATP. Thus CD39 positive lymphoma cells exert an ATPase activity and might suppress antitumor responses.

### Example 7: Inhibition of lymphoma-CD39 enzymatic activity by BA54g, BY12 and BY40

Protocol: 1x10⁵ lymphoma cells (Ramos) were cultured in complete RPMI medium alone or in presence of BA54g, BY12 or BY40 mAbs, at the indicated concentrations, for 16h. Cells were then washed three times in phosphate free buffer (10 mM glucose, 20 mM Hepes, 5 mM KCl, 120 mM NaCl, 2 mM CaCl2) and resuspended in the same buffer supplemented with 100 µM ATP for 15 min. Phosphate concentration in supernatants was measured after addition of Malachite green/polyvinylalcohol /ammonium mobylate solution for 20 min by a spectrophotometer at 620 nm and compared against a standard curve. Results are the mean of two independent experiments.

Results: **Inhibition of lymphoma-CD39 enzymatic activity by CD39 mAbs BA54g, BY12 and BY40.** In order to test whether CD39 mAbs might be useful to inhibit CD39-mediated immunosupression by lymphoma cells we cultured the CD39 positive Ramos cell line with increasing doses of 3 CD39 mAbs. As shown in figure 7, the three antibodies BA54g, BY12 and BY40 inhibited CD39 activity in a dose dependant manner. Altogether these results demonstrate that CD39 is expressed and active at the surface of some cancer cells (e.g., melanoma and lymphoma). CD39 activity participates to the generation of adenosine which leads to the decreased response of the immune system with an inhibition of the antitumor response. In conclusion we show here that CD39 mAbs BA54g, BY12 and BY40 are able to decrease CD39 enzymatic activity and are thus promising tools to block tumor-CD39 mediated immunosuppression.

### Example 8: Expression and activity of CD39 on ovarian cancer cells.

Protocol: (**A**) Ovarian cancer cells (OAW42) were stained with CD39 antibody (clone A1, Ebiosciences) and CD39 expression was analysed by flow cytometry. Results are representative of two independent experiments. (**B**) 5x10⁴ OAW42 cells were cultured in complete RPMI medium for 30 min with 10 µM ATP. The concentration of "unhydrolysed" extracellular ATP was determined using the ATPlite luminescence ATP Detection Assay System (Perkin Elmer). Experiment was performed in triplicate.

Results: **Ovarian cancer cells express functional CD39.** In order to assess whether ovarian cancer cells might also expressed CD39, we measured CD39 expression at the cell surface of the ovarian cancer cell line OAW42 by flow cytometry using the CD39 mAb A1 (Ebiosciences). As shown in figure 8, OAW42 cells expressed CD39 at their cell surface. Furthermore, we measured their capacity to hydrolyze ATP by measuring ATP remaining in cell supernatant after culture of cells with ATP. As shown in figure 8, OAW42 cells degraded about 60% of ATP after 30 min of culture. Thus, ovarian cancer cells express a functional CD39 enzyme and might thus participate to suppress antitumor responses through the degradation of ATP and generation of adenosine.

### Example 9: Expression of CD39 on a panel of normal and cancer tissues.

Protocol: Multiple organ cancer and normal tissue Microarray (MC5002) from US Biomax, Inc. were used to analyze expressions of CD39. MC5002 contained 500 cores with 18 of most common types of cancer (20 -25 cases/type) and normal controls (5 cases/type). The tissues were formalin fixed, paraffin embedded. Array sections were mounted on the positive charged Fisher SuperFrost glass slide which is suitable for antigen detection. IHC staining was performed using CD39 antibody at 1.52 µg/mL (clone 22A9, Abcam). Manual scoring of intensity of staining was performed. The intensity (Strength, 0-3) of CD39 staining was scored as negative (0), possible positive (0.5), weak (1), moderate (2), or strong (3). Results for normal or cancer tissues from lymph node (**A**), thyroid (**B**), lung (**C**) or kidney (**D**) are shown. Each dot represents one sample.

Results: **CD39 is expressed by tumor cells in various human cancers.** In order to assess CD39 expression in a large panel of cancer, we analyzed CD39 expression in cancer and normal tissue Microarray. As shown on Figure 9, CD39 staining scored as moderate to strong on many cancer tissues including lymphoma cells or carcinoma from thyroid lung or kidney. For these tissues, CD39 staining is higher in cancer samples compared to that of normal tissues.

## Claims

1. A CD39 antagonistic antibody for use for treating cancer by inhibiting the immunosuppressive effects of a CD39-expressing cancerous cell, wherein said cancer is selected from the group consisting of ovarian cancer, thyroid cancer, lung cancer, and kidney cancer.

2. The CD39 antagonistic antibody for use according to claim 1, wherein said cancerous cell is selected from ovarian cancer, thyroid cancer, lung cancer, and kidney cancer.

3. The CD39 antagonistic antibody for use according to claim 1 or 2, wherein said antagonist is an antibody able to down modulate the cell membrane expression of CD39 and/or to block or decrease CD39 ATPase/ADPase activity and/or to block or decrease cancer cells-mediated inhibition or suppression of the immune antitumor response.

4. The CD39 antagonistic antibody for use according to claim 3, wherein said antagonist is a CD39 monoclonal antibody.

5. The CD39 antagonistic antibody for use according to claim 4, wherein said antibody comprises a variable light chain (VL) comprising the CDRs of the VL chain of the antibody obtainable from hybridoma deposited as CNCM-I-3889 and a variable heavy chain (VH) comprising the CDRs of the VH chain of the antibody obtainable from hybridoma deposited as CNCM-I-3889.

6. The CD39 antagonistic antibody for use according to claim 4, wherein said antibody comprises:
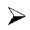 a heavy chain wherein the variable domain comprises at least one CDR having a sequence selected from the group consisting of SEQ ID NO:2 for CDR-H1, SEQ ID NO:3 for CDR-H2 and SEQ ID NO:4 for CDR-H3; and/or
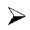 a light chain wherein the variable domain comprises at least one CDR having a sequence selected from the group consisting of SEQ ID NO:6 for CDR-L1, SEQ ID NO:7 for CDR-L2 and SEQ ID NO:8 for CDR-L3.

7. The CD39 antagonistic antibody for use according to claim 4, wherein said antibody comprises a variable light chain (VL) comprising the CDRs of the VL chain of the antibody obtainable from hybridoma deposited as CNCM-I-4171 and a variable heavy chain (VH) comprising the CDRs of the VH chain of the antibody obtainable from hybridoma deposited as CNCM-I-4171.

8. The CD39 antagonistic antibody for use according to claim 4, wherein said antibody comprises:
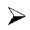 a heavy chain wherein the variable domain comprises at least one CDR having a sequence selected from the group consisting of SEQ ID NO:12 for CDR-H1, SEQ ID NO:13 for CDR-H2 and SEQ ID NO:14 for CDR-H3; and/or
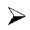 a light chain wherein the variable domain comprises at least one CDR having a sequence selected from the group consisting of SEQ ID NO:16 for CDR-L1, SEQ ID NO:17 for CDR-L2 and SEQ ID NO:18 for CDR-L3.

9. The CD39 antagonistic antibody for use according to claim 4, wherein said antibody is selected from the group consisting of:
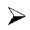 the antibody produced by the hybridoma cell line deposited with the CNCM on January 4, 2008 under the accession number 1-3889; and
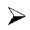 the antibody produced by the hybridoma cell line deposited with the CNCM on June 23, 2009 under the accession number CNCM 1-4171.

## Patentansprüche

1. Ein CD39 antagonistischer Antikörper zur Verwendung zur Behandlung von Krebs durch Hemmung der immunsuppressiven Effekte einer CD39-exprimierenden Krebszelle, wobei der Krebs ausgewählt ist aus der Gruppe bestehend aus Eierstockkrebs, Schilddrüsenkrebs, Lungenkrebs und Nierenkrebs.

2. Der CD39 antagonistische Antikörper zur Verwendung nach Anspruch 1, wobei die Krebszelle ausgewählt ist aus Eierstockkrebs, Schilddrüsenkrebs, Lungenkrebs und Nierenkrebs.

3. Der CD39 antagonistische Antikörper nach Anspruch 1 oder 2, wobei der Antagonist ein Antikörper ist, der in der Lage ist, die Zellmembranexpression von CD39 herunter zu modulieren und/oder die CD39 ATPase/ADPase Aktivität zu blockieren oder zu reduzieren und/oder die Krebszell vermittelte Hemmung oder Unterdrückung der Immun-Antitumor-Antwort zu blockieren oder zu reduzieren.

4. Der CD39 antagonistische Antikörper zur Verwendung nach Anspruch 3, wobei der Antagonist ein CD39 monoklonaler Antikörper ist.

5. Der CD39 antagonistische Antikörper zur Verwendung nach Anspruch 4, wobei der Antikörper eine variable leichte Kette (VL), umfassend die CDRs der VL-Kette des Antikörpers erhältlich von der Hybridomzellline hinterlegt als CNCM-I-3889, und eine variable schwere Kette (VH), umfassend die CDRs der VH-Kette des Antikörpers erhältlich von der Hybridomzellline hinterlegt als CNCM-I-3889, umfasst.

6. Der CD39 antagonistische Antikörper zur Verwendung nach Anspruch 4, wobei der Antikörper umfasst:
- eine schwere Kette, wobei der variable Bereich mindestens eine CDR mit einer Sequenz ausgewählt aus der Gruppe bestehend aus SEQ ID NO:2 für CDR-H1, SEQ ID NO:3 für CDR-H2 und SEQ ID NO:4 für CDR-H3 umfasst; und/oder
- eine leichte Kette, wobei der variable Bereich mindestens eine CDR mit einer Sequenz ausgewählt aus der Gruppe bestehend aus SEQ ID NO:6 für CDR-L1, SEQ ID NO:7 für CDR-L2 und SEQ ID NO:8 für CDR-L3 umfasst.

7. Der CD39 antagonistische Antikörper zur Verwendung nach Anspruch 4, wobei der Antikörper eine variable leichte Kette (VL), umfassend die CDRs der VL-Kette des Antikörpers erhältlich von der Hybridomzellline hinterlegt als CNCM-I-4171, und eine variable schwere Kette (VH), umfassend die CDRs der VH-Kette des Antikörpers erhältlich von der Hybridomzellline hinterlegt als CNCM-I-4171, umfasst.

8. Der CD39 antagonistische Antikörper zur Verwendung nach Anspruch 4, wobei der Antikörper umfasst:
- eine schwere Kette, wobei der variable Bereich mindestens eine CDR mit einer Sequenz ausgewählt aus der Gruppe bestehend aus SEQ ID NO:12 für CDR-H1, SEQ ID NO:13 für CDR-H2 und SEQ ID NO:14 für CDR-H3 umfasst; und/oder
- eine leichte Kette, wobei der variable Bereich mindestens eine CDR mit einer Sequenz ausgewählt aus der Gruppe bestehend aus SEQ ID NO:16 für CDR-L1, SEQ ID NO:17 für CDR-L2 und SEQ ID NO:18 für CDR-L3 umfasst.

9. Der CD39 antagonistische Antikörper zur Verwendung nach Anspruch 4, wobei der Antikörper ausgewählt ist aus der Gruppe bestehend aus:
- dem Antikörper hergestellt von der Hybridomzellline hinterlegt bei der CNCM am 4. Januar 2008 unter der Zugangsnummer I-3889; und
- dem Antikörper hergestellt von der Hybridomzellline hinterlegt bei der CNCM am 23. Juni 2009 unter der Zugangsnummer I-4171.

## Revendications

1. Anticorps antagoniste du CD39 destiné à une utilisation pour le traitement du cancer par inhibition des effets immunosuppresseurs d'une cellule cancéreuse exprimant le CD39, dans lequel ledit cancer est choisi dans le groupe constitué d'un cancer ovarien, un cancer de la thyroïde, un cancer du poumon, et un cancer du rein.

2. Anticorps antagoniste du CD39 destiné à une utilisation selon la revendication 1, dans lequel ladite cellule cancéreuse est choisie parmi un cancer ovarien, un cancer de la thyroïde, un cancer du poumon, et un cancer du rein.

3. Anticorps antagoniste du CD39 destiné à une utilisation selon la revendication 1 ou 2, dans lequel ledit antagoniste est un anticorps capable de moduler à la baisse l'expression par la membrane cellulaire du CD39 et/ou de bloquer ou de diminuer l'activité ATPase/ADPase du CD39 et/ou de bloquer ou de diminuer l'inhibition ou la suppression médiée par les cellules cancéreuses de la réponse immunitaire antitumorale.

4. Anticorps antagoniste du CD39 destiné à une utilisation selon la revendication 3, dans lequel ledit antagoniste est un anticorps monoclonal anti-CD39.

5. Anticorps antagoniste du CD39 destiné à une utilisation selon la revendication 4, dans lequel ledit anticorps comprend une chaîne légère variable (VL) comprenant les CDR de la chaîne VL de l'anticorps pouvant être obtenu à partir de l'hybridome déposé sous la référence CNCM-I-3889 et une chaîne lourde variable (VH) comprenant les CDR de la chaîne VH de l'anticorps pouvant être obtenu à partir de l'hybridome déposé sous la référence CNCM-I-3889.

6. Anticorps antagoniste du CD39 destiné à une utilisation selon la revendication 4, dans lequel ledit anticorps comprend :
- une chaîne lourde dans laquelle le domaine variable comprend au moins une CDR ayant une séquence choisie dans le groupe constitué de SEQ ID NO : 2 pour la CDR-H1, SEQ ID NO : 3 pour la CDR-H2 et SEQ ID NO : 4 pour la CDR-H3 ; et/ou
- une chaîne légère dans laquelle le domaine variable comprend au moins une CDR ayant une séquence choisie dans le groupe constitué de SEQ ID NO : 6 pour la CDR-L1, SEQ ID NO : 7 pour la CDR-L2 et SEQ ID NO : 8 pour la CDR-L3.

7. Anticorps antagoniste du CD39 destiné à une utilisation selon la revendication 4, dans lequel ledit anticorps comprend une chaîne légère variable (VL) comprenant les CDR de la chaîne VL de l'anticorps pouvant être obtenu à partir de l'hybridome déposé sous la référence CNCM-I-4171 et une chaîne lourde variable (VH) comprenant les CDR de la chaîne VH de l'anticorps pouvant être obtenu à partir de l'hybridome déposé sous la référence CNCM-I-4171.

8. Anticorps antagoniste du CD39 destiné à une utilisation selon la revendication 4, dans lequel ledit anticorps comprend :
- une chaîne lourde dans laquelle le domaine variable comprend au moins une CDR ayant une séquence choisie dans le groupe constitué de SEQ ID NO : 12 pour la CDR-H1, SEQ ID NO : 13 pour la CDR-H2 et SEQ ID NO : 14 pour la CDR-H3 ; et/ou
- une chaîne légère dans laquelle le domaine variable comprend au moins une CDR ayant une séquence choisie dans le groupe constitué de SEQ ID NO : 16 pour la CDR-L1, SEQ ID NO : 17 pour la CDR-L2 et SEQ ID NO : 18 pour la CDR-L3.

9. Anticorps antagoniste du CD39 destiné à une utilisation selon la revendication 4, dans lequel ledit anticorps est choisi dans le groupe constitué de :
- l'anticorps produit par la lignée cellulaire d'hybridome déposée à la CNCM le 4 janvier 2008 sous le numéro d'accession I-3889 ; et
- l'anticorps produit par la lignée cellulaire d'hybridome déposée à la CNCM le 23 juin 2009 sous le numéro d'accession CNCM I-4171.


### SEQUENCE LISTING

<110> OREGA BIOTECH
<120> ANTIBODIES AGAINST HUMAN CD39 AND USE THEREOF
<130>
<160> 20
<170> PatentIn version 3.5
<210> 1
   <211> 187
   <212> PRT
   <213> Mus musculus
<400> 1
   Thr Arg Val Lys Lys Pro Arg Glu Thr Val Lys Ile Ser Cys Lys Ala
   1               5                   10                  15
   Ser Gly Tyr Thr Phe Thr His Tyr Gly Met Asn Trp Val Lys Gln Ala
               20                  25                  30
   Pro Gly Lys Gly Leu Lys Trp Met Gly Trp Ile Asn Thr Tyr Thr Gly
           35                  40                  45
   Glu Pro Thr Tyr Ala Asp Asp Phe Lys Gly Arg Phe Ala Phe Ser Leu
       50                  55                  60
   Glu Ala Ser Val Ser Thr Ala Tyr Leu Gln Ile Asn Asn Leu Lys Asn
   65                  70                  75                  80
   Glu Asp Thr Ala Thr Tyr Phe Cys Ala Arg Arg Arg Tyr Glu Gly Asn
                   85                  90                  95
   Tyr Val Phe Tyr Tyr Phe Asp Tyr Trp Gly Gln Gly Thr Thr Leu Thr
               100                 105                 110
   Val Ser Ser Ala Lys Thr Thr Pro Pro Ser Val Tyr Pro Leu Ala Pro
           115                 120                 125
   Gly Ser Ala Ala Gln Thr Asn Ser Met Val Thr Leu Gly Cys Leu Val
       130                 135                 140
   Lys Gly Tyr Phe Pro Glu Gln Val Thr Val Thr Trp Asn Ser Gly Ser
   145                 150                 155                 160
   Leu Ser Ser Gly Val His Thr Phe Pro Ala Val Leu Gln Ser Asp Leu
                   165                 170                 175
   Tyr Thr Leu Ser Ser Ser Val Thr Val Pro Ser
               180                 185
<210> 2
   <211> 8
   <212> PRT
   <213> Mus musculus
<400> 2
   Gly Tyr Thr Phe Thr His Tyr Gly
   1               5
<210> 3
   <211> 8
   <212> PRT
   <213> Mus musculus
<400> 3
   Ile Asn Thr Tyr Thr Gly Glu Pro
   1               5
<210> 4
   <211> 27
   <212> PRT
   <213> Mus musculus
<400> 4
   Ala Arg Arg Arg Tyr Glu Gly Asn Tyr Val Phe Tyr Tyr Phe Asp Tyr
   1               5                   10                  15
   Trp Gly Gln Gly Thr Thr Leu Thr Val Ser Ser
               20                  25
<210> 5
   <211> 165
   <212> PRT
   <213> Mus musculus
<400> 5
   Asp Ile Gln Met Thr Gln Ser Pro Ala Ser Leu Ser Ala Ser Val Gly
   1               5                   10                  15
   Glu Thr Val Thr Ile Thr Cys Arg Ala Ser Glu Asn Ile Tyr Ser Tyr
               20                  25                  30
   Phe Ser Trp Tyr Gln Gln Lys Gln Gly Lys Ser Pro Gln Leu Leu Val
           35                  40                  45
   Tyr Thr Ala Lys Thr Leu Ala Glu Gly Val Pro Ser Arg Phe Ser Gly
       50                  55                  60
   Ser Gly Ser Gly Thr Gln Phe Ser Leu Lys Ile Asn Ser Leu Gln Pro
   65                  70                  75                  80
   Glu Asp Phe Gly Ser Tyr Tyr Cys Gln His His Tyr Val Thr Pro Tyr
   85 90 95
   Thr Phe Gly Gly Gly Thr Lys Leu Glu Ile Lys Arg Ala Asp Ala Ala
   100 105 110
   Pro Thr Val Ser Ile Phe Pro Pro Ser Ser Glu Gln Leu Thr Ser Gly
   115 120 125
   Gly Ala Ser Val Val Cys Phe Leu Asn Asn Phe Tyr Pro Lys Asp Ile
   130 135 140
   Asn Val Lys Trp Lys Ile Asp Gly Ser Glu Arg Gln Asn Gly Val Leu
   145 150 155 160
   Asn Ser Trp Thr Asp
   165
<210> 6
   <211> 11
   <212> PRT
   <213> Mus musculus
<400> 6
   Arg Ala Ser Glu Asn Ile Tyr Ser Tyr Phe Ser
   1 5 10
<210> 7
   <211> 7
   <212> PRT
   <213> Mus musculus
<400> 7
   Thr Ala Lys Thr Leu Ala Glu
   1 5
<210> 8
   <211> 20
   <212> PRT
   <213> Mus musculus
<400> 8
   Gln His His Tyr Val Thr Pro Tyr Thr Phe Gly Gly Gly Thr Lys Leu
   1 5 10 15
   Glu Ile Lys Arg
   20
<210> 9
   <211> 345
   <212> DNA
   <213> Mus musculus
<400> 9
   acgcgagtga agaagcctcg agagacagtc aagatctcct gcaaggcttc tgggtatacc      60
   ttcacacact atggaatgaa ctgggtgaag caggctccag gaaagggttt aaagtggatg     120
   ggctggataa acacctacac tggagagcca acatatgctg atgacttcaa gggacggttt     180
   gccttctctt tggaagcctc tgtcagcact gcctatttgc agatcaacaa cctcaaaaat     240
   gaggacacgg ctacatattt ctgtgcaaga aggagatatg agggtaacta cgttttttac     300
   tactttgact actggggcca aggcaccact ctcacagtct cctca                     345
<210> 10
   <211> 324
   <212> DNA
   <213> Mus musculus
<400> 10
   gacatccaga tgactcagtc tccagcctcc ctatctgcat ctgtgggaga aactgtcacc      60
   atcacatgtc gagcaagtga aaatatttac agttattttt catggtatca gcagaaacag     120
   ggaaaatctc ctcagctcct ggtctatact gcaaaaacct tagcagaagg tgtgccatca     180
   aggttcagtg gcagtggatc aggcacacag ttttctctga agatcaacag cctgcagcct     240
   gaagattttg ggagttatta ctgtcaacat cattatgtta ctccgtacac gttcggaggg     300
   gggaccaagc tggaaataaa acgg                                            324
<210> 11
   <211> 118
   <212> PRT
   <213> Mus musculus
<400> 11
   Asp Val Gln Leu Val Glu Ser Gly Gly Gly Leu Val Gln Pro Gly Gly
   1               5                   10                  15
   Ser Arg Lys Leu Ser Cys Ala Ala Ser Gly Phe Thr Phe Ser Ser Phe
               20                  25                  30
   Gly Met His Trp Val Arg Gln Ala Pro Glu Lys Gly Leu Glu Trp Val
           35                  40                  45
   Ala Tyr Ile Ser Ser Gly Ser Ser Ile Ile Tyr Tyr Ala Asp Thr Val
       50                  55                  60
   Lys Gly Arg Phe Thr Ile Ser Arg Asp Asn Pro Lys Asn Thr Leu Phe
   65                  70                  75                  80
   Leu Gln Met Thr Ser Leu Gly Ser Glu Asp Thr Ala Met Tyr Tyr Cys
                   85                  90                  95
   Ala Arg Trp Ser Thr Thr Val Val Ala Thr Asp Tyr Trp Gly Gln Gly
               100                 105                 110
   Thr Thr Leu Thr Val Ser
           115
<210> 12
   <211> 21
   <212> PRT
   <213> Mus musculus
<400> 12
   Gly Gly Ser Arg Lys Leu Ser Cys Ala Ala Ser Gly Phe Thr Phe Ser
   1               5                   10                  15
   Ser Phe Gly Met His
               20
<210> 13
   <211> 17
   <212> PRT
   <213> Mus musculus
<400> 13
   Tyr Ile Ser Ser Gly Ser Ser Ile Ile Tyr Tyr Ala Asp Thr Val Lys
   1               5                   10                  15
   Gly
<210> 14
   <211> 20
   <212> PRT
   <213> Mus musculus
<400> 14
   Trp Ser Thr Thr Val Val Ala Thr Asp Tyr Trp Gly Gln Gly Thr Thr
   1               5                   10                  15
   Leu Thr Val Ser
               20
<210> 15
   <211> 108
   <212> PRT
   <213> Mus musculus
<400> 15
   Asn Ile Val Met Thr Gln Ser Pro Lys Ser Met Ser Met Ser Val Gly
   1               5                   10                  15
   Glu Arg Val Thr Leu Thr Cys Lys Ala Ser Glu Asn Val Val Thr Tyr
               20                  25                  30
   Val Ser Trp Tyr Gln Gln Lys Pro Glu Gln Ser Pro Lys Leu Leu Ile
           35                  40                  45
   Tyr Gly Ala Ser Asn Arg Tyr Thr Gly Val Pro Asp Arg Phe Thr Gly
       50                  55                  60
   Ser Gly Ser Ala Thr Asp Phe Thr Leu Thr Ile Ser Ser Val Gln Ala
   65                  70                  75                  80
   Glu Asp Leu Ala Asp Tyr His Cys Gly Gln Gly Tyr Ser Tyr Pro Tyr
                   85                  90                  95
   Thr Phe Gly Gly Gly Thr Lys Leu Glu Ile Lys Arg
               100                 105
<210> 16
   <211> 11
   <212> PRT
   <213> Mus musculus
<400> 16
   Lys Ala Ser Glu Asn Val Val Thr Tyr Val Ser
   1               5                   10
<210> 17
   <211> 7
   <212> PRT
   <213> Mus musculus
<400> 17
   Gly Ala Ser Asn Arg Tyr Thr
   1 5
<210> 18
   <211> 21
   <212> PRT
   <213> Mus musculus
<400> 18
   Cys Gly Gln Gly Tyr Ser Tyr Pro Tyr Thr Phe Gly Gly Gly Thr Lys
   1 5 10 15
   Leu Glu Ile Lys Arg
   20
<210> 19
   <211> 354
   <212> DNA
   <213> Mus musculus
<400> 19
   gatgtgcagc tggtggagtc tgggggaggc ttagtgcagc ctggagggtc ccggaaactc 60
   tcctgtgcag cctctggatt cactttcagt agctttggaa tgcactgggt tcgtcaggct 120
   ccagagaagg ggctggagtg ggtcgcatac attagtagtg gcagtagtat tatctactat 180
   gcagacacag tgaagggccg attcaccatc tccagagaca atcccaagaa caccctgttc 240
   ctgcaaatga ccagtctagg gtctgaggac acggccatgt attactgtgc aagatggagt 300
   actacggtag tagctacaga ctactggggc caaggcacca ctctcacagt ctcc 354
<210> 20
   <211> 324
   <212> DNA
   <213> Mus musculus
<400> 20
   aacattgtaa tgacccaatc tcccaaatcc atgtccatgt cagtaggaga gagggtcacc      60
   ttgacctgca aggccagtga gaatgtggtt acttatgttt cctggtatca acagaaacca     120
   gagcagtctc ctaaactgct gatatacggg gcatccaacc ggtacactgg ggtccccgat     180
   cgcttcacag gcagtggatc tgcaacagat ttcactctga ccatcagcag tgtgcaggct     240
   gaagaccttg cagattatca ctgtggacag ggttacagct atccgtacac gttcggaggg     300
   gggaccaagc tggaaataaa acgg                                            324